(19) Europäisches Patentamt
European Patent Office
Office européen des brevets

(11) Numéro de publication : **0 286 515 B1**

(12) # FASCICULE DE BREVET EUROPEEN

(45) Date de publication du fascicule du brevet : **29.04.92 Bulletin 92/18**

(51) Int. Cl.$^5$ : **C07D 307/92,** C07D 307/93, C07D 409/12, A61K 31/34

(21) Numéro de dépôt : **88400779.0**

(22) Date de dépôt : **31.03.88**

(54) **Nouvelles amines tricycliques dérivées du tétrahydro-5,6,7,8 naphto [2,3b] dihydro-2,3 furanne, et du tétrahydro-6,7,8,9 5H-benzocyclohepta [2,3b] dihydro-2,3 furanne, leurs procédés de préparation et les compositions pharmaceutiques qui les contiennent.**

(30) Priorité : **01.04.87 FR 8704550**

(43) Date de publication de la demande : **12.10.88 Bulletin 88/41**

(45) Mention de la délivrance du brevet : **29.04.92 Bulletin 92/18**

(84) Etats contractants désignés : **AT BE CH DE FR GB IT LI LU NL SE**

(56) Documents cités :
**GB-A- 1 084 439**
**US-A- 4 470 990**
**CHIMIE THERAPEUTIQUE, vol. 6, no. 3, mai-juin 1971, pages 196-202, Paris, FR; R. VIOLLAND et al.: "VIII. Psychotropes potentiels. Synthèse et activité pharmacologique d'amino-2 tétralines apparentées à divers psychotomimétiques"**

(73) Titulaire : **ADIR ET COMPAGNIE**
**1 rue Carle Hébert**
**F-92415 Courbevoie Cédex (FR)**

(72) Inventeur : **Peglion, Jean-Louis**
**5 allée des Bégonias**
**F-78110 Le Vésinet (FR)**
Inventeur : **Poignant, Jean-Claude La Guyonnerie**
**13 rue de la Fontaine St-Mathieu**
**F-91440 Bures sur Yvette (FR)**
Inventeur : **Vian, Joel**
**12 avenue Ste-Marie**
**F-92370 Chaville (FR)**

EP 0 286 515 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles amines tricycliques dérivées du tétrahydro-5,6,7,8 naphto [2,3b] dihydro-2,3 furanne et du tétrahydro-6,7,8,9 5H-benzocyclohepta [2,3b] dihydro-2,3 furanne, leurs procédés de préparation et les compositions pharmaceutiques qui les contiennent.

On connait certains composés tricycliques dérivés de l'amino tétrahydro naphtalène pharmacologiquement actifs. En effet, quelques dérivés de la tétrahydro-7,8,9,10 benzo [h] quinolyle-9 amine, dont l'activité antidépressive n'a été évaluée que par des essais in vitro, sont mentionnés dans la littérature (Brevet U.S. 4,521,423). Des tétrahydro-6,7,8,9 benzo [g] indolyl-8 amines et des tétrahydro-6,7,8,9 naphto [1,2b] furannyl-8 amines douées d'une activité stimulante dopaminergique sont décrites dans les brevets U.S. 4,510,157 et U.S. 4,470,990.

La demanderesse a maintenant découvert que certaines amines tricycliques dérivées du tétrahydro-5,6,7,8 naphto [2,3b] dihydro-2,3 furanne ou du tétrahydro-6,7,8,9 5H-benzocyclohepta [2,3b] dihydro-2,3 furanne de structure originale, possèdent des propriétés pharmacologiques très intéressantes. En effet, les composés de la présente invention possèdent des propriétés dopaminergiques et une activité antidépressive, antiagressive et psychostimulante importante prouvée par des essais in vivo.

La présente invention a plus particulièrement pour objet les dérivés de formule générale I,

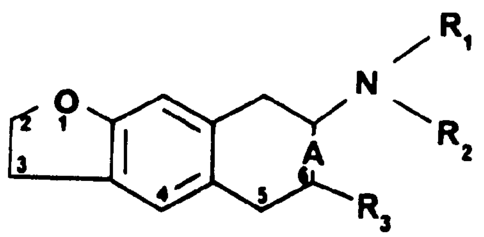

(I)

dans laquelle,

- $R_1$ et $R_2$, identiques ou différents, représentent chacun :
  - un atome d'hydrogène,
  - un radical benzyle,
  - un radical cyclohexylméthyle,
  - un radical alkyle, linéaire ou ramifié renfermant de 1 à 10 atomes de carbone éventuellement substitué par un radical hydroxy, un radical carboxy, un radical alcoxy de 1 à 5 atomes de carbone, ou un radical alcoxycarbonyle de 2 à 6 atomes de carbone,
  - un radical phénylalkyle de 7 à 16 atomes de carbone, ou
  - un radical thiényl-2-alkyle de 5 à 14 atomes de carbone ;
- $R_3$ représente un atome d'hydrogène ou un radical alkyle linéaire ou ramifié renfermant de 1 à 4 atomes de carbone,
- A représente une simple liaison ou un radical méthylène, ou un radical de formule,

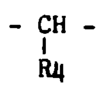

dans laquelle $R_4$ est un radical alkyle linéaire ou ramifié renfermant de 1 à 4 atomes de carbone, sous forme racémique ou d'isomères optiques,
et leurs sels d'addition à un acide minéral ou organique, pharmaceutiquement acceptable.

La présente invention a également pour objet le procédé de préparation des composés de formule générale I, caractérisé en ce que

l'on condense le dihydro-2,3 benzofuranne de formule II,

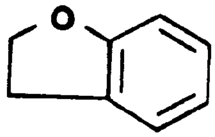

(II)

en présence d'un solvant organique chloré et de chlorure d'aluminium avec un dérivé de l'anhydride aspartique ou glutamique de formule générale III,

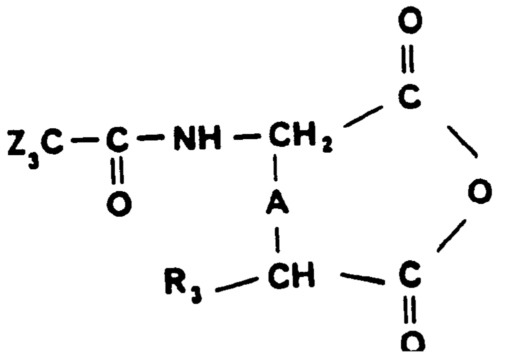

(III)

dans laquelle la signification de A et $R_3$ est identique à celle donnée pour la formule I et Z représente un atome d'hydrogène ou de fluor, pour former les composés de formule générale IV,

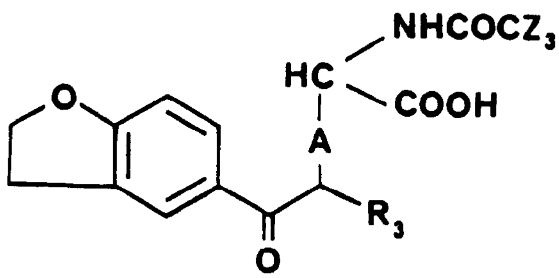

(IV)

dans laquelle $R_3$, A et Z ont la signification indiquée ci-dessus,
que l'on réduit à chaud par le triéthylsilane en présence d'acide trifluoroacétique pour former les composés de formule générale V,

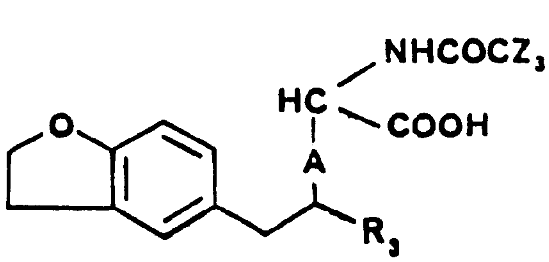

(V)

dans laquelle $R_3$, A et Z ont la signification précédemment indiquée,
que l'on soumet à chaud à l'action du pentoxyde de phosphore en présence d'acide phosphorique pour former les composés de formule générale VI,

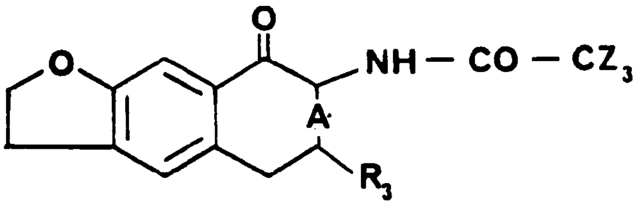

(VI)

dans laquelle la signification de $R_3$, Z et A est celle indiquée ci-dessus, que l'on soumet à une hydrogénation catalytique à la température ambiante en milieu acide et en présence de palladium sur charbon à 5% pour obtenir un amide de formule générale VII,

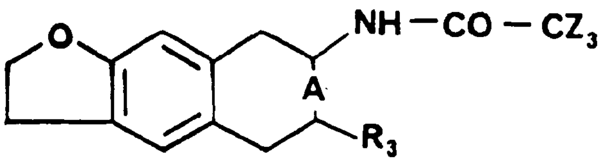

(VII)

dans laquelle $R_3$, A et Z ont la signification précédemment indiquée, que l'on fait réagir avec une base forte pour obtenir un composé de formule générale I dans laquelle $R_1$ et $R_2$ identiques représentent chacun un atome d'hydrogène,
lequel ensuite, que l'on peut alcoyler pour former les amines secondaires ou tertiaires correspondantes,

. ou en le condensant avec un composé de formule générale VIII,

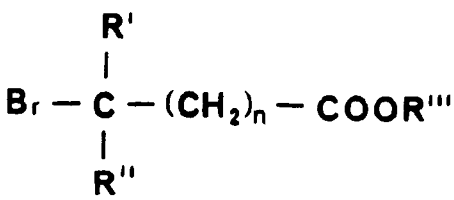

(VIII)

dans laquelle R′ et R″ identiques ou différents représentent chacun un atome d'hydrogène ou un radical alkyle inférieur de 1 à 4 atomes de carbone, R‴ représente un radical alkyle inférieur de 1 à 5 atomes de carbone et n est un nombre entier de 0 à 9, pour former un composé de formule générale I,
dans laquelle $R_1$ représente un atome d'hydrogène et $R_2$ représente un radical alkyle linéaire ou ramifié renfermant de 1 à 10 atomes de carbone substitué par un radical alcoxycarbonyle de 2 à 6 atomes de carbone,
lequel ensuite que l'on peut soumettre à l'action d'une base minérale forte pour former un composé de formule générale I,
dans laquelle $R_1$ représente un hydrogène et $R_2$ représente un radical alkyle de 1 à 10 atomes de carbone linéaire ou ramifié substitué par un radical carboxy,

. ou en le faisant réagir avec un chlorure d'acide de formule générale IX,

$$W(CH_2)_nCOCl \quad (IX)$$

dans laquelle n est un nombre entier de 0 à 9 et W représente un radical phényle ou un radical thiényle-2, et ensuite en réduisant le composé issu de cette réaction par un hydrure métallique double pour former un composé de formule générale I dans laquelle $R_1$ représente un radical alkyle de 1 à 10 atomes de carbone substitué par un radical phényle ou un radical thiényle-2, et $R_2$ est un hydrogène,

. ou en le faisant réagir avec une quantité adéquate de formaldéhyde et de l'acide formique pour obtenir les composés de la formule générale I, dans laquelle $R_3$ et $R_4$ identiques représentent chacun un radical méthyle,

. ou en le faisant réagir avec un iodure d'alkyle de formule générale X,

$$IR \quad (X)$$

dans laquelle R représente un radical alkyle linéaire ou ramifié renfermant de 1 à 10 atomes de carbone (éventuellement substitué par un radical hydroxy ou par un radical alcoxy de 1 à 5 atomes de carbone) ou un radical cyclohexylméthyle, à chaud dans un solvant organique en présence d'une base minérale,
pour former les composés de formule générale I, dans laquelle $R_1$ et $R_2$ identiques ont la même signification que R,

. ou en le soumettant à l'action du benzaldéhyde à chaud et en présence d'un alcool de petit poids moléculaire, pour former la benzylimine correspondante, et ensuite, à une hydrogénation catalytique, et puis à l'action de l'acide formique et du formaldéhyde, pour former les composés de formule générale I dans laquelle $R_1$ représente un radical méthyle et $R_2$ un radical cyclohexylméthyle,

. ou en le soumettant d'abord à l'action du benzaldéhyde en présence d'un solvant aromatique inerte et apolaire et ensuite, après élimination du solvant utilisé, à l'action du borohydrure de sodium, en présence d'un alcool aliphatique polaire de petit poids moléculaire, pour obtenir un composé de formule générale I, dans laquelle $R_1$ représente un hydrogène et $R_2$ un radical benzyle,

lequel ensuite,
que l'on peut soumettre :

. soit à l'action du formaldéhyde et de l'acide formique pour former les composés de formule générale I, dans laquelle $R_1$ représente un radical méthyle et $R_2$ un radical benzyle,

. soit à l'action d'un iodure d'alkyle de formule générale X, pour former les composés de formule générale I dans laquelle $R_1$ a la même signification que R et $R_2$ représente un radical benzyle,

lequel ensuite,
que l'on peut soumettre à une hydrogénation catalytique pour former les composés de formule générale I dans laquelle $R_1$ a la signification donnée ci-dessus et $R_2$ représente un atome d'hydrogène,
lequel ensuite,
que l'on peut soumettre à l'action d'un iodure d'alkyle de formule générale X pour former les composés de formule générale I dans laquelle $R_1$ et $R_2$ identiques ou différents représentent chacun un radical alkyle renfermant de 1 à 10 atomes de carbone (éventuellement substitué par un radical hydroxy ou un radical alcoxy de 1 à 5 atomes de carbone) ou un radical cyclohexylméthyle,
que l'on peut ensuite, si on le désire, salifier avec un acide minéral ou organique pharmaceutiquement acceptable,
ou séparer en leurs isomères optiques et ensuite salifier.

Les différents composés de formule générale III sont obtenus en faisant réagir l'anhydride acétique ou l'anhydride trifluoroacétique avec les acides aspartique, glutamique ou leurs analogues substitués par un alkyle en β ou γ. La réaction est réalisée d'abord à une température inférieure à zéro et ensuite à une température comprise entre 30 et 60°C, selon la méthode décrite par BARKER C.C. dans J.Chem.Soc. (1953),p.453.

Les dérivés de l'acide aspartique comportant une chaîne alkyle en bêta sont obtenus selon la méthode décrite par DAKIN H.D. dans J.Biol.Chem. (1941),141,p.945:950.

Les méthodes de synthèse des dérivés de l'acide glutamique substitués par un alkyle en β ou γ sont aussi connues. (Gershon H, Parmegiani R, Giannasio V et Krull J., J.Pharm.Scien.,(1975),64,N°11,p. 1855-1858).

Les composés de formule générale I peuvent être séparés en leurs isomères optiques après formation des sels avec les acides (d) et (l) camphosulfonique.

Parmi les acides pharmaceutiquement acceptables pour la préparation des sels d'addition aux composés de formule générale I, on peut citer les acides chlorhydrique, phosphorique, fumarique, citrique, oxalique, sulfurique, tartrique, maléïque, mandélique, méthanesulfonique etc...

Les composés selon l'invention, ainsi que leurs sels et leurs isomères optiques sont doués de propriétés pharmacologiques fort intéressantes.

En effet, les essais pharmacologiques in vivo ont montré que les composés possèdent de puissantes propriétés antidépressives, antiagressives et psychostimulantes. Ces propriétés ont été mises en évidence au moyen d'essais classiquement utilisés chez l'animal permettant de présager avec une très bonne précision l'activité chez l'homme. ("Antidepressants: Neurochemical Behavioral and Clinical Perspectives" Enna S.J., Malick J., Richelson E., Raven Press Ed., 1981, N.Y. et "Industrial Pharmacology, Antidepressants II", Fielding Stuart, Harbans Lal, Futura Publishing Comp. Ed., 1975, N.Y.).

Les effets antiagressifs des composés de l'invention ont été recherchés par deux méthodes. La première a permis d'évaluer l'inhibition des conduites agressives chez la souris préalablement isolée (Charpentier J. "Analysis and measurement of aggressive behaviour in mice", Agressive Behaviour, Garattini S. and Sigg E.B. Ed., p.86-100, Excerpta Medica Found. Amsterdam, 1969), et la deuxième, l'inhibition de l'agressivité du rat isolé et bulbectomisé (Karli P, Vergnes M., and Didiergeorges F, "Rat mouse interspecific aggressive behaviour and its manipulation by brain ablation and by brain stimulation", Agressive Behaviour, Garattini S. and Sigg. E.B. Ed., p 47-55, Excerpta Medica Found. Amsterdam, 1969).

Les composés de l'invention inhibent l'agressivité d'isolement chez la souris et l'agressivité du rat bulbectomisé isolé. Les dose efficaces sont comprises entre 3 et 5 $mg.kg^{-1}$ par voie intrapéritonéale.

Les effets antidépresseurs des composés de l'invention ont été recherchés en mettant en oeuvre les méthodes de l'antagonisme de l'hypothermie induite par la réserpine et de l'antagonisme des ondes ponto-géniculo-occipitales induites par le composé Ro4-1284 chez le chat.

Les essais pour l'évaluation de l'antagonisme de l'hypothermie induite par la réserpine ont été réalisés sur des souris mâles Swiss CD. Après répartition des animaux en groupes, la réserpine a été injectée par voie intrapéritonéale à la dose de 2,5 $mg.kg^{-1}$. Trois heures plus tard, les composés de l'invention ont été administrés par la même voie. La température rectale des animaux a été mesurée 1 heure et 2 heures après le second traitement et comparée à la température initiale de ces mêmes animaux, mesurée immédiatement avant l'administration des composés soumis à l'essai. Au temps de mesure 2 heures après l'administration, les composés de l'invention, à la dose de 2,5 $mg.kg^{-1}$ i.p., antagonisent à 60% environ l'hypothermie induite par la réserpine.

Pour l'évaluation de l'antagonisme des ondes ponto-géniculo-occipitales P.G.O. induites par le Ro4-1284 chez le chat, la méthode décrite par Ruch-Monachon M.A., Jalfre M., et Haefely W (Arch.Int.Pharm.Therap., (1976),219, N°2,p. 251-346) a été utilisée.

Les composés de l'invention antagonisent fortement les ondes P.G.O. provoquées chez le chat par le composé Ro4-1284 dépléteur des monoamines cérébrales. Les doses efficaces (i.v.) inhibant de 50% le taux des ondes P.G.O. ($DE_{50}$) pour certains composés de l'invention soumis à cet essai sont de l'ordre de 0,14 à 0,20 $mg.kg^{-1}$.

Les résultats des études pharmacologiques ont mis en évidence que les composés de l'invention ont une action dopaminergique, et plus spécialement une activité dopaminostimulante. Cette activité a été évaluée par l'épreuve de discrimination de drogue chez le rat dont les principes généraux sont évoqués dans "Drug discrimination, application in C.N.S. Pharmacology", F. Colpaert Ed., Elsevier Biomedical, 1982, Amsterdam. A la dose de 2,5 $mg.kg^{-1}$ administrée par voie intrapéritonéale, les composés de l'invention se comportent comme l'apomorphine sur le test de discrimination de drogue, ce qui prouve leur action dopaminergique de nature agoniste. Cette activité est plus marquée quand sont utilisés pour l'épreuve des isomères dextrogyres des composés de l'invention. Récemment, il a été constaté que, lors de la maladie de Parkinson, il existe une déplétion en dopamine des noyaux gris centraux du système extrapyramidal. C'est ainsi que les agonistes dopaminergiques, comme les composés de la présente invention, peuvent avoir des effets thérapeutiques très intéressants pour le traitement symptomatique de cette maladie. (Burgers Medicinal Chemistry 4th Ed.,Part III, p. 413-430,(1981) J. Wiley and Sons Edt.) La dopamine exerce aussi un effet puissamment freinateur de la sécrétion de prolactine, hormone ayant comme principale action le développement et le maintien de la lactogénèse. Les composés de la présente invention peuvent donc être utilisés aussi pour le traitement des troubles neuroendocriniens dus à un déficit dopaminergique tels que hyperprolactinémie et galactorrhée. (The Pharmacological basis of Therapeutics,7th Ed., p. 1374-1385, Goodman Gilman A. Ed., Macmillan Publish. Comp. NY).

L'invention s'étend aussi aux compositions pharmaceutiques renfermant comme principe actif au moins un composé de formule générale I, l'un de ses isomères ou l'un de ses sels avec un acide minéral ou organique pharmaceutiquement compatible, en association avec un ou plusieurs excipients inertes, et appropriés.

Les compositions pharmaceutiques ainsi obtenues sont présentées avantageusement sous des formes diverses telles que par exemple comprimés, dragées, gélules, glossettes ou autres préparations galéniques appropriées pour une admininstration sublinguale, suppositoires, solutions injectables ou buvables.

La posologie peut varier largement en fonction de l'âge, du poids du patient, de la nature et de la sévérité de l'affection ainsi que de la voie d'administration.

La voie d'administration préférée est la voie orale ou parentérale.

D'une manière générale, la posologie unitaire s'échelonnera entre 0,5 et 100 mg et la posologie journalière, utilisable en thérapeutique humaine, entre 10 et 100 mg.

Les exemples suivants, donnés à titre non-limitatif, illustrent l'invention.

Les points de fusion sont mesurés selon la technique Micro-Köfler. Les spectres de résonance magnétique nucléaire du proton (R.M.N.) ont été enregistrés à 60 MHz.

## EXEMPLE 1

### Anhydride N-trifluoroacetyl aspartique

Refroidir à -10°C, 0,49 mole d'acide aspartique et ajouter goutte à goutte 1,23 mole d'anhydride trifluoroacétique en maintenant la température à -10°C. Laisser revenir le milieu réactionnel à température ambiante et porter progressivement à reflux pendant 2 heures. Refroidir, agiter en présence d'hexane et filtrer. Sécher le résidu solide obtenu.

Rendement : 99%

Point de fusion : 137-138°C

**EXEMPLE 2**

**(d.1) amino-7 tétrahydro-5,6,7,8 naphto [2,3b] dihydro-2,3 furanne**

STADE A

Acide [dihydro-2,3 benzofurannyl-5]-4 oxo-4 trifluoroacétamido-2 butanoïque

Dans une solution de 12 ml de dichloroéthane contenant 0,05 mole de dihydro-2,3 benzofuranne, ajouter 0,1 mole du composé obtenu à l'exemple 1 et 0,05 mole de chlorure d'aluminium en suspension dans 100 ml de dichloroéthane. Agiter pendant 36 heures à température ambiante et ensuite hydrolyser avec une solution saturée de chlorure d'ammonium. Extraire par le dichlorométhane, laver à l'eau, sécher sur sulfate de magnésium, filtrer et concentrer pour obtenir le produit attendu.
Rendement : 88%
Point de fusion : 160-162°C

STADE B

Acide [dihydro-2,3 benzofurannyl-5]-4 trifluoroacétamido-2 butanoïque

Dans une solution de 97 ml d'acide trifluoroacétique contenant 0,063 mole du composé obtenu au stade précédent, introduire 0,25 ml de triéthylsilane. Porter à reflux pendant deux heures et ensuite verser le milieu réactionnel sur de la glace. Extraire à l'éther. Laver la phase organique à l'eau et ensuite la sécher sur sulfate de magnésium anhydre. Concentrer sous pression réduite (0,5 mm/Hg). Sécher sous vide sur pentoxyde de phosphore et hydroxyde de potassium.
Cristalliser dans un mélange de chloroforme, toluène et hexane (20:20:80 V/V), pour obtenir l'acide [dihydro-2,3 benzofurannyl-5]-4 trifluoroacétamido-2 butanoïque pur.
Rendement : 82%
Point de fusion : 125°C

STADE C

Trifluoroacétamido-7 tétrahydro-5,6,7,8 naphto [2,3b] dihydro-2,3 furannone-8

Ajouter dans 456 ml d'acide phosphorique à 80%, 712 g de pentoxyde de phosphore. Après chauffage pendant 2 heures à 100°C, laisser la température revenir à 60°C et ajouter 0,09 mole d'acide [dihydro-2,3 benzofurannyl-5]-4 trifluoroacétamido-2 butanoïque obtenu au stade précédent. Agiter fortement le milieu réactionnel pendant une heure, puis refroidir et le verser sur de l'eau contenant de la glace. Extraire à l'éther. Laver la phase organique par une solution aqueuse de bicarbonate de sodium à 10% et ensuite par une solution saturée de chlorure de sodium. La trifluoroacétamido-7 tétrahydro-5,6,7,8 naphto [2,3b] dihydro-2,3 furannone-8 est obtenue après séchage de la phase éthérée sur sulfate de sodium anhydre et évaporation sous pression réduite. Le composé est ensuite recristallisé dans l'acétonitrile.
Rendement : 40%
Point de fusion : 208°C

STADE D

Trifluoroacétamido-7 tétrahydro-5,6,7,8 naphto [2,3b] dihydro-2,3 furanne

Dissoudre 0,07 mole environ du composé obtenu au stade précédent dans un mélange de 100 ml d'acide acétique et de 2 ml d'acide perchlorique à 70%. Réduire sous pression de 5 kg/cm$^2$ d'hydrogène en présence de 1 g de palladium sur charbon à 5%, ensuite filtrer et concentrer sous pression réduite. Reprendre à l'eau. Séparer la phase aqueuse, sécher la phase organique sur sulfate de magnésium anhydre et concentrer. Recristalliser dans l'éther isopropylique pour obtenir le trifluoroacétamido-7 tétrahydro-5,6,7,8 naphto [2,3b] dihydro-2,3 furanne pur.
Rendement : 55%

Point de fusion : 131°C

STADE E

L'amide obtenue au stade précédent est porté à reflux pendant 18 heures avec 25 ml d'acide chlorhydrique 4N. Après refroidissement, le milieu réactionnel est alcalinisé et extrait par le dichlorométhane. Après séchage et évaporation, on obtient l'amino-7 tétrahydro-5,6,7,8 naphto [2,3b] dihydro-2,3 furanne.
Point de fusion : 60°C

Après dissolution de l'amine ainsi obtenue dans l'acétate d'éthyle, additionner lentement et sous agitation une quantité adéquate d'éther chlorhydrique pour obtenir le chlorhydrate correspondant.
Point de fusion : 275°C
Spectre RMN ($CDCl_3$ + DMSO-$d_6$) : 1,7 à 3,7 ppm,m, 9H; 4,6 ppm,t,2H; 6,5 ppm,m,1H; 7 ppm,m,1H; 8,65 ppm,3H échangeables.

**EXEMPLE 3**

**amino-7 méthyl-6 tétrahydro-5,6,7,8 naphto [2,3b] dihydro-2,3 furanne**

Ce composé peut être préparé selon le procédé décrit dans l'exemple 2 mais, en utilisant au stade A, l'anhydride méthyl-3 N-trifluoroacétyl aspartique. Ce dernier composé peut être préparé selon le procédé décrit dans l'exemple 1. La préparation de l'acide correspondant est décrit dans J.Biol.Chem.,(1941),141,p.945-950.

**EXEMPLE 4**

**amino-8 tétrahydro-6,7,8,9 5H-benzocyclohepta [2,3b] dihydro-2,3 furanne**

Ce composé peut être préparé selon le procédé décrit dans l'exemple 2 mais, en utilisant au stade A, l'anhydride N-trifluoroacétyl glutamique au lieu de l'anhydride N-trifluoroacétyl aspartique.

**EXEMPLE 5**

**amino-8 méthyl-7 tétrahydro-6,7,8,9 5H-benzocyclohepta [2,3b] dihydro-2,3 furanne**

Ce composé peut être préparé à partir de l'anhydride méthyl-3 N-trifluoroacétyl glutamique et selon le procédé décrit dans l'exemple 2. L'acide méthyl-3 glutamique nécessaire pour la synthèse de l'anhydride mentionné ci-dessus peut être préparé selon le procédé décrit dans J.Pharm.Scien.,(1975),64,N°11,p.1855-1858.

**EXEMPLE 6**

**amino-8 méthyl-6 tétrahydro-6,7,8,9 5H-benzocyclohepta [2,3b] dihydro-2,3 furanne**

Ce composé peut être préparé en condensant l'anhydride méthyl-4 N-trifluoroacétyl glutamique avec le dihydro-2,3 benzofuranne selon le procédé décrit dans l'exemple 2. La synthèse de l'acide méthyl-4 glutamique est connue (J.Pharm.Scien.,(1975),64,N°11,p.1855-1858).

**EXEMPLE 7**

**Camphosulfonate de (d) amino-7 tétrahydro-5,6,7,8 naphto [2,3b] dihydro-2,3 furanne**

Ce sel a été obtenu en faisant réagir le composé de l'exemple 2 avec une quantité équimolaire de l'acide (d) camphosulfonique. Après deux recristallisations dans l'éthanol suivies de deux recristallisations dans le méthanol, le sel est obtenu optiquement pur.
(Estimation par H.P.L.C. : > 99%)
Point de fusion : 253-261°C
Pouvoir rotatoire d'une solution à 0,5% dans l'eau :

| λ nm | $[\alpha]_D^{23°C}$ |
|---|---|
| 589 | + 41,3° |
| 578 | + 43,3° |
| 546 | + 51,1° |
| 436 | + 105,6° |
| 365 | + 235,0° |

**EXEMPLE 8**

**Camphosulfonate de (1) amino-7 tétrahydro-5,6,7,8 naphto [2,3b] dihydro-2,3 furanne**

Ce composé a été obtenu selon le procédé décrit dans l'exemple 7 mais en utilisant l'acide (1) camphosulfonique.
Point de fusion : 254-265°C
Pouvoir rotatoire d'une solution à 0,5% dans l'eau :

| λ nm | $[\alpha]_D^{23°C}$ |
|---|---|
| 589 | - 49,1° |
| 578 | - 51,4° |
| 546 | - 60,3° |
| 436 | - 125,0° |
| 365 | - 276,4° |

**EXEMPLE 9**

Chlorhydrate de (d) amino-7 tétrahydro-5,6,7,8 naphto [2,3b] dihydro-2,3 furanne

Le composé de l'exemple 7 est mis en solution dans l'acétate d'éthyle et ensuite le milieu est basifié par l'hydroxyde de sodium. La phase organique est séparée, séchée sur sulfate de sodium anhydre et évaporée. L'huile obtenue est reprise dans l'acétonitrile, et ensuite une quantité stoechiométrique d'éther chlorhydrique est additionnée, pour obtenir le chlorhydrate de (d) amino-7 tétrahydro-5,6,7,8 naphto [2,3b] dihydro-2,3 furanne optiquement pur.
Point de fusion : 261-264°C
Pouvoir rotatoire d'une solution à 0,25% dans le D.M.S.O. :

| λ nm | $[\alpha]_D^{23°C}$ |
|---|---|
| 589 | + 86,4° |
| 578 | + 90,4° |
| 546 | + 104,4° |
| 436 | + 191,6° |
| 365 | + 350,2° |

**EXEMPLE 10**

**Chlorhydrate de (1) amino-7 tétrahydro-5,6,7,8 naphto [2,3b] dihydro-2,3 furanne**

Ce composé a été obtenu à partir du composé de l'exemple 8 et selon le procédé décrit dans l'exemple 9.
Point de fusion : 262-264°C
Pouvoir rotatoire d'une solution à 0,25% dans le D.M.S.O. :

| λ nm | $[\alpha]_D^{23°C}$ |
|---|---|
| 589 | - 86,4° |
| 578 | - 90,4° |
| 546 | - 104,4° |
| 436 | - 191,6° |
| 365 | - 350,2° |

**PREPARATION PHARMACEUTIQUE**

**EXEMPLE 11**

**Comprimés dosés à 25 mg de chlorhydrate de (d) amino-7 tétrahydro-5,6,7,8 naphto [2,3b] dihydro-2,3 furanne**

| | |
|---|---|
| Chlorhydrate de (d) amino-7 tétrahydro-5,6,7,8 naphto [2,3b] dihydro-2,3 furanne | 25,00 g |
| amidon de blé | 100,00 g |
| amidon de maïs | 80,00 g |
| stéarate de magnésium | 15,00 g |
| talc | 20,00 g |

pour 1000 comprimés à 25 mg de principe actif.

## Revendications

1. Composés de formule générale I,

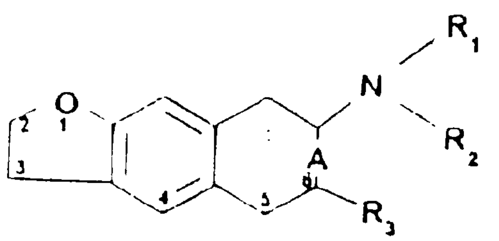

(I)

dans laquelle :

– $R_1$ et $R_2$ identiques ou différents représentent chacun : un atome d'hydrogène, un radical benzyle, un radical cyclohexylméthyle, un radical alkyle, linéaire ou ramifié renfermant de 1 à 10 atomes de carbone éventuellement substitué par un radical hydroxy, par un radical carboxy, un radical alcoxy de 1 à 5 atomes de carbone, ou un radical alcoxycarbonyl de 2 à 6 atomes de carbone, un radical alkyl phényle de 7 à 16 atomes de carbone, ou un radical thiényl-2-alkyle de 5 à 14 atomes de carbone ;

– $R_3$ représente un atome d'hydrogène ou un radical alkyle linéaire ou ramifié renfermant de 1 à 4 atomes de carbone,

– A représente une simple liaison ou un radical méthylène, ou un radical de formule,

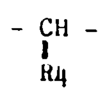

dans laquelle $R_4$ représente un radical alkyle linéaire ou ramifié renfermant de 1 à 4 atomes de carbone,
à condition toutefois que, quand A représente une simple liaison, R3, R1 et R2 représentent chacun un atome d'hydrogène,
sous forme racémique ou d'isomères optiques,
et leurs sels d'addition à un acide minéral ou organique, pharmaceutiquement acceptable.

2. Composés de formule générale I selon la revendication 1 dans laquelle A représente une simple liaison.

3. Composés de formule générale I selon la revendication 1 dans laquelle A représente un radical méthylène.

4. Procédé de préparation des composés de formule générale I, caractérisé en ce que l'on condense le dihydro-2,3 benzofuranne de formule II,

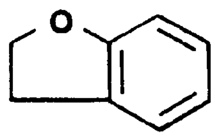

(II)

en présence d'un solvant organique chloré et de chlorure d'aluminium avec un dérivé de l'anhydride aspartique ou glutamique de formule générale III

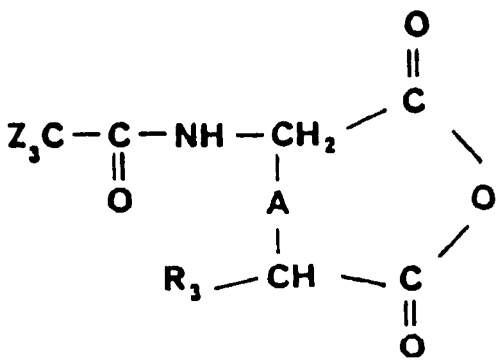

(III)

dans laquelle la signification de A et $R_3$ est identique à celle donnée pour la formule I et Z représente un atome d'hydrogène ou de fluor, pour former les composés de formule générale IV,

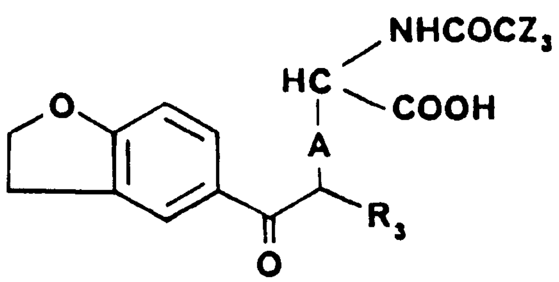

(IV)

dans laquelle $R_3$, A et Z ont la signification indiquée ci-dessus,
que l'on réduit à chaud par le triéthylsilane en présence d'acide trifluoroacétique pour former les composés de formule générale V,

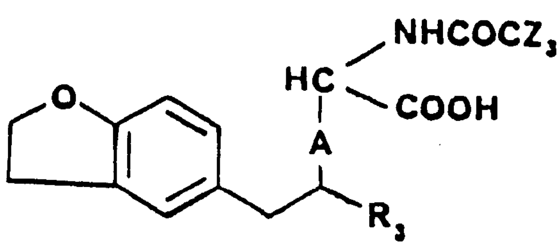

(V)

dans laguelle $R_3$, A et Z ont la signification précédemment indiquée,
que l'on soumet à chaud à l'action du pentoxyde de phosphore en présence d'acide phosphorique pour former les composés de formule générale VI,

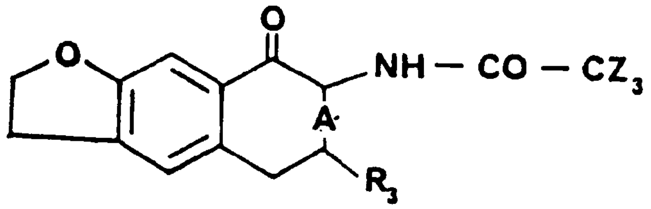

(VI)

dans laquelle la signification de $R_3$, Z et A est celle indiquée ci-dessus,
que l'on soumet à une hydrogénation catalytique à la température ambiante en milieu acide et en présence de palladium sur charbon à 5% pour obtenir un amide de formule générale VII,

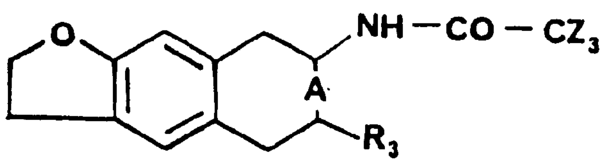

(VII)

dans laquelle $R_3$, A et Z ont la signification précédemment indiquée,
que l'on fait réagir avec une base forte pour obtenir un composé de formule générale I dans laquelle $R_1$ et $R_2$ identiques représentent chacun un atome d'hydrogène,
lequel ensuite, que l'on peut alcoyler pour former les amines secondaires ou tertiaires correspondantes,

. ou en le condensant avec un composé de formule générale VIII,

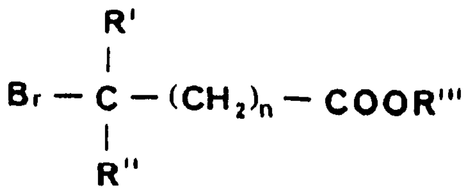

(VIII)

dans laquelle R′ et R″ identiques ou différents représentent chacun un atome d'hydrogène ou un radical allyle inférieur de 1 à 4 atomes de carbone, R‴ représente un radical allyle inférieur de 1 à 5 atomes de carbone et n est un nombre entier de 0 à 9, pour former un composé de formule générale I,
dans laquelle $R_1$ représente un atome d'hydrogène et $R_2$ représente un radical allyle linéaire ou ramifié renfermant de 1 à 10 atomes de carbone substitué par un radical alcoxycarbonyle de 2 à 6 atomes de carbone,
lequel ensuite que l'on peut soumettre à l'action d'une base minérale forte pour former un composé de formule générale I, dans laquelle $R_1$ représente un atome d'hydrogène et $R_2$ représente un radical allyle de 1 à 10 atomes de carbone linéaire ou ramifié substitué par un radical carboxy,

. ou en le faisant réagir avec un chlorure d'acide de formule générale IX,

$$W(CH_2)_nCOCl \quad (IX)$$

dans laquelle n est un nombre entier de 0 à 9 et W représente un radical phényle ou un radical thiényle-2, et ensuite en réduisant le composé issu de cette réaction par un hydrure métallique double pour former un composé de formule générale I dans laquelle $R_1$ représente un radical alkyle de 1 à 10 atomes de carbone substitué par un radical phényle ou un radical thiényle-2, et $R_2$ est un hydrogène,

. ou en le faisant réagir avec une quantité adéquate de formaldéhyde et de l'acide formique pour obtenir les composés de la formule générale I, dans laquelle $R_3$ et $R_4$ identiques représentent chacun un radical méthyle,

. ou en le faisant réagir avec un iodure d'alkyle de formule générale X,

$$IR \quad (X)$$

dans laquelle R représente un radical allyle linéaire ou ramifié renfermant de 1 à 10 atomes de carbone (éventuellement substitué par un radical hydroxy ou par un radical alcoxy de 1 à 5 atomes de carbone) ou un radical cyclohexylméthyle, à chaud dans un solvant organique en présence d'une base minérale,

pour former les composés de formule générale I, dans laquelle $R_1$ et $R_2$ identiques ont la même signification que R,

. ou en le soumettant à l'action du benzaldéhyde à chaud et en présence d'un alcool de petit poids moléculaire, pour former la benzylimine correspondante, et ensuite, à une hydrogénation catalytique, et puis à l'action de l'acide formique et du formaldéhyde, pour former les composés de formule générale I dans laquelle $R_1$ représente un radical méthyle et $R_2$ un radical cyclohexylméthyle,

. ou en le soumettant d'abord à l'action du benzaldéhyde en présence d'un solvant aromatique inerte et apolaire et ensuite, après élimination du solvant utilisé, à l'action du borohydrure le sodium, en présence d'un alcool aliphatique polaire de petit poids moléculaire, pour obtenir un composé de formule générale I, dans laquelle $R_1$ représente un hydrogène et $R_2$ un radical benzyle,

lequel ensuite,

que l'on peut soumettre:

. soit à l'action du formaldéhyde et de l'acide formique pour former les composés de formule générale I, dans laquelle $R_1$ représente un radical méthyle et $R_2$ un radical benzyle,

. soit à l'action d'un iodure d'alkyle de formule générale X, pour former les composés de formule générale I dans laquelle $R_1$ a la même signification que R et $R_2$ représente un radical benzyle,

lequel ensuite,

que l'on peut soumettre à une hydrogénation catalytique pour former les composés de formule générale I dans laquelle $R_1$ a la signification donnée ci-dessus et R2 représente un atome d'hydrogène,

lequel ensuite,

que l'on peut soumettre à l'action d'un iodure d'alkyle de formule générale X pour former les composés de formule générale I dans laquelle $R_1$ et $R_2$ identiques ou différents représentent chacun un radical allyle renfermant de 1 à 10 atomes de carbone (éventuellement substitué par un radical hydroxy ou un radical alcoxy de 1 à 5 atomes de carbone) ou un radical cyclohexylméthyle,

que l'on peut ensuite, si on le désire, salifier avec un acide minéral ou organique pharmaceutiquement acceptable,

ou séparer en leurs isomères optiques et ensuite salifier.

5. Composition pharmaceutique contenant comme principe actif un composé selon l'une quelconque des revendications 1 à 3, en association ou en mélange avec un excipient ou un véhicule inerte non toxique, pharmaceutiquement acceptable.

6. Composition pharmaceutique selon la revendication 5 renfermant le principe actif à la dose de 0,5 à 100 mg.

7. Composition pharmaceutique selon les revendications 5 et 6 renfermant comme principe actif au moins un composé selon les revendications 1 à 3, utilisable dans le traitement des maladies nécessitant des médications antidépressives, antiagressives ou des modulateurs dopaminergiques.

8. Composés de formule générale IV,

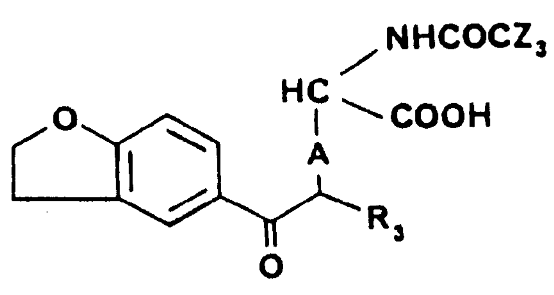

IV

dans laquelle

– $R_3$ représente un atome d'hydrogène ou un radical alkyle linéaire ou ramifié renfermant de 1 à 4 atomes de carbone,

– A représente une simple liaison ou un radical méthylène, ou un radical de formule

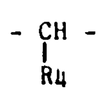

dans laquelle ,

– $R_4$ représente un radical alkyle linaire ou ramifié renfermant de 1 à 4 atomes de carbone,

et

– Z représente un atome d'hydrogène ou un atome de fluor,

utiles comme intermédiaires à la préparation des composés de formule générale I selon la revendication 1.

9. Composés de formule générale V,

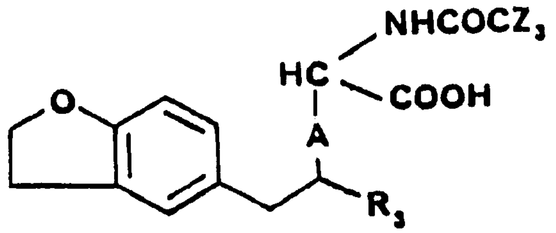

V

dans laquelle,

– $R_3$ représente un atome d'hydrogène ou un radical allyle linéaire ou ramifié renfermant de 1 à 4 atomes de carbone,

– A représente une simple liaison ou un radical méthylène, ou un radical de formule,

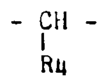

dans laquelle,

– $R_4$ représente un radical alkyle linéaire ou ramifié renfermant de 1 à 4 atomes de carbone,

et

– Z représente un atome d'hydrogène ou un atome de fluor,

utiles comme intermédiaires à la préparation des composés de formule générale I selon la revendication 1.

## Claims

1. Compounds of the general formula I

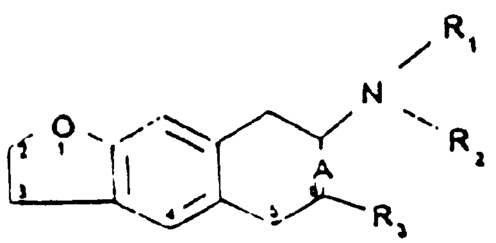

(I)

in which :

– $R_1$ and $R_2$, which may be the same or different, each represents a hydrogen atom, a benzyl radical, a cyclohexylmethyl radical or a linear or branched alkyl radical containing from 1 to 10 carbon atoms that is optionally substituted by a hydroxy radical, by a carboxy radical or by an alkoxy radical containing from 1 to 5 carbon atoms, by an alkoxycarbonyl radical containing from 2 to 6 carbon atoms, by a alkylphenyl radical containing from 7 to 16 carbon atoms or by a alkylthien-2-yl radical containing from 5 to 14 carbon atoms,

– $R_3$ represents a hydrogen atom or a linear or branched alkyl radical containing from 1 to 4 carbon atoms,

– A represents a single bond or a methylene radical or a radical of the formula

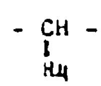

in which $R_4$ represents a linear or branched alkyl radical containing from 1 to 4 carbon atoms,

with the proviso, however, that when A represents a single bond $R_3$, $R_1$ and $R_2$ each represents a hydrogen

atom,
in racemic form or in the form of optical isomers,
and their addition salts with a pharmaceutically acceptable organic or mineral acid.

2. Compounds of the general formula I according to claim 1, in which A represents a single bond.

3. Compounds of the general formula I according to claim 1, in which A represents a methylene radical.

4. Process for the preparation of compounds of the general formula I, characterised in that the 2,3-dihydrobenzofuran of formula II

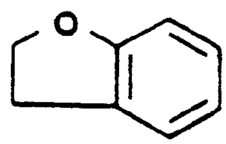

(II)

is condensed in the presence of a chlorinated organic solvent and aluminium chloride with a glutamic or aspartic anhydride compound of the general formula III

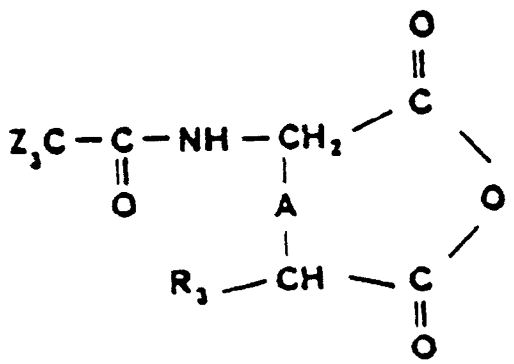

(III)

in which the meaning of A and $R_3$ is identical to that given for formula I and Z represents a hydrogen or fluorine atom, to form a compound of the general formula IV

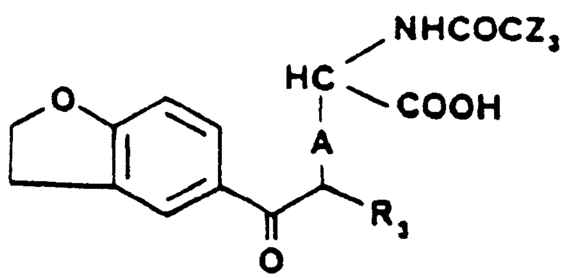

(IV)

in which $R_3$, A and Z are as defined above,
which is reduced with triethylsilane with the application of heat in the presence of trifluoroacetic acid to yield a compound of the general formula V

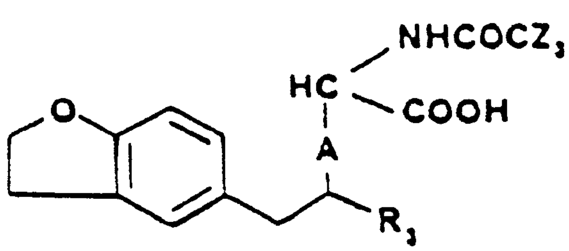

(V)

in which $R_3$, A and Z are as defined above,
which is subjected, with the application of heat, to the action of phosphorus pentoxide in the presence of phosphoric acid to form a compound of the general formula VI

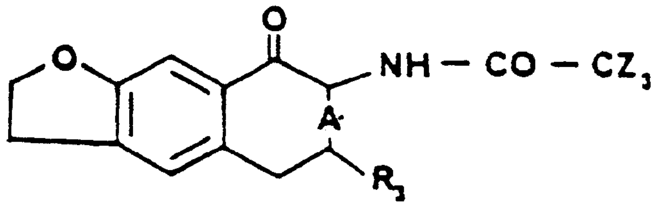

(VI)

in which $R_3$, Z and A are as defined above,
which is subjected to catalytic hydrogenation at room temperature in acid medium and in the presence of 5 % palladium-on-carbon to yield an amide of the general formula VII

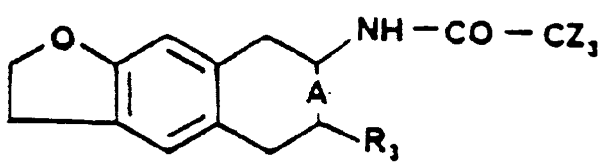

(VII)

in which $R_3$, A and Z are as defined above,
which is reacted with a strong base to obtain a compound of the general formula I in which $R_1$ and $R_2$, which are the same, each represents a hydrogen atom,
which then may be alkylated to form a corresponding secondary or tertiary amine,

. either by condensing with a compound of the general formula VIII

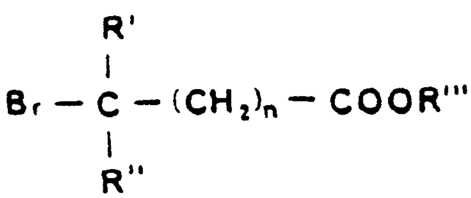

(VIII)

in which R′ and R″, which may be the same or different, each represents a hydrogen atom or a lower alkyl radical containing from 1 to 4 carbon atoms, R‴ represents a lower alkyl radical containing from 1 to 5 carbon atoms and n is an integer of from 0 to 9, to yield a compound of the general formula I,
in which $R_1$ represents a hydrogen atom and $R_2$ represents a linear or branched alkyl radical containing from 1 to 10 carbon atoms that is substituted by an alkoxycarbonyl radical containing from 2 to 6 carbon atoms, which may then be subjected to the action of a strong mineral base to yield a compound of the general formula I in which $R_1$ represents a hydrogen atom and $R_2$ represents a linear or branched alkyl radical containing from 1 to 10 carbon atoms that is substituted by a carboxy radical,

. or by reacting with an acid chloride of the general formula IX

$$W(CH_2)_nCOCl \quad (IX)$$

in which n is an integer of from 0 to 9 and W represents a phenyl radical or a 2-thienyl radical, and then reducing the compound obtained by this reaction with a double metal hydride to yield a compound of the general formula I in which $R_1$ represents an alkyl radical containing from 1 to 10 carbon atoms that is substituted by a phenyl radical or by a 2-thienyl radical, and $R_2$ is a hydrogen atom,

. or by reacting with an appropriate amount of formaldehyde and formic acid to obtain a compound of the general formula I in which $R_3$ and $R_4$, which are the same, each represents a methyl radical,

. or by reacting with an alkyl iodide of the general formula X

$$IR \quad (X)$$

in which R represents a linear or branched alkyl radical containing from 1 to 10 carbon atoms (optionally substituted by a hydroxy radical or by an alkoxy radical containing from 1 to 5 carbon atoms) or a cyclohexylmethyl radical, with the application of heat in an organic solvent in the presence of a mineral base
to yield a compound of the general formula I in which $R_1$ and $R_2$, which are the same, have the same meaning

as R,

. or by subjecting to the action of benzaldehyde, with the application of heat and in the presence of a low molecular weight alcohol, to yield the corresponding benzylimine, and then to catalytic hydrogenation and subsequently to the action of formic acid and formaldehyde to yield a compound of the general formula I in which $R_1$ represents a methyl radical and $R_2$ represents a cyclohexylmethyl radical,

. or subjecting first to the action of benzaldehyde in the presence of an inert non-polar aromatic solvent and then, after removal of the solvent used, to the action of sodium borohydride in the presence of a low molecular weight polar aliphatic alcohol, to obtain a compound of the general formula I in which $R_1$ represents a hydrogen atom and $R_2$ represents a benzyl radical,

which then

may be subjected:

. either to the action of formaldehyde and formic acid to yield a compound of the general formula I in which $R_1$ represents a methyl radical and $R_2$ represents a benzyl radical,

. or to the action of an alkyl iodide of the general formula X to yield a compound of the general formula I in which $R_1$ has the same meaning as R and $R_2$ represents a benzyl radical,

which then

may be subjected to catalytic hydrogenation to yield a compound of the general formula I in which $R_1$ is as defined above and $R_2$ represents a hydrogen atom,

which then

may be subjected to the action of an alkyl iodide of the general formula X to yield a compound of the general formula I in which $R_1$ and $R_2$, which may be the same or different, each represents an alkyl radical containing from 1 to 10 carbon atoms (optionally substituted by a hydroxy radical or an alkoxy radical containing from 1 to 5 carbon atoms) or a cyclohexylmethyl radical,

which may then, if desired, be converted into a salt with a pharmaceutically acceptable organic or mineral acid,

or separated into its optical isomers and then converted into salt form.

5. Pharmaceutical composition containing as active ingredient a compound according to any one of claims 1 to 3 in association or in admixture with a pharmaceutically acceptable non-toxic inert vehicle or excipient.

6. Pharmaceutical composition according to claim 5 containing the active ingredient in an amount of from 0.5 to 100 mg.

7. Pharmaceutical composition according to claims 5 and 6 containing as active ingredient at least one compound according to claims 1 to 3 which can be used in the treatment of disorders necessitating anti-depressant or anti-aggression medication or dopaminergic modulators.

8. Compounds of the general formula IV

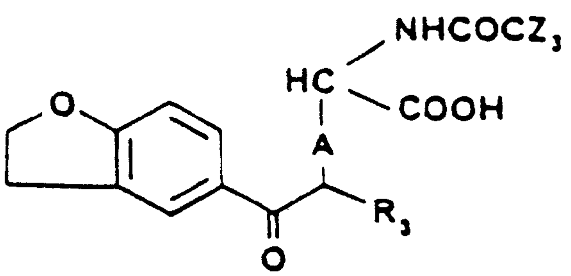

IV

in which

– $R_3$ represents a hydrogen atom or a linear or branched alkyl radical containing from 1 to 4 carbon atoms,

– A represents a single bond or a methylene radical, or a radical of the formula

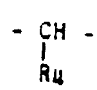

in which

– $R_4$ represents a linear or branched alkyl radical containing from 1 to 4 carbon atoms,

and

– Z represents a hydrogen atom or a fluorine atom,

which can be used as intermediates in the preparation of compounds of the general formula I according to claim 1.

9. Compounds of the general formula V

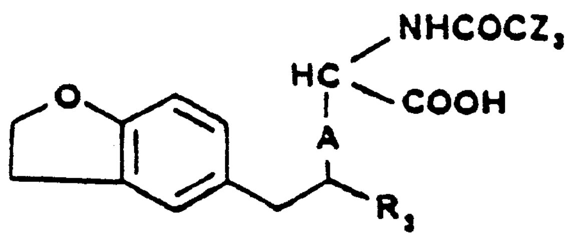

V

in which

– $R_3$ represents a hydrogen atom or a linear or branched alkyl radical containing from 1 to 4 carbon atoms,

– A represents a single bond or a methylene radical, or a radical of the formula

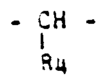

in which

– $R_4$ represents a linear or branched alkyl radical containing from 1 to 4 carbon atoms,

and

– Z represents a hydrogen atom or a fluorine atom,

which can be used as intermediates in the preparation of compounds of the general formula I according to claim 1.

## Patentansprüche

1. Verbindungen der allgemeinen Formel (I)

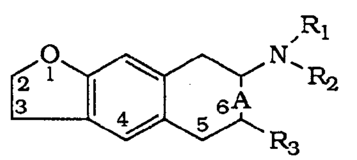

(I)

in der

– $R_1$ und $R_2$, die gleichartig oder verschieden sein können, jeweils Wasserstoffatome, Benzylgruppen, Cyclohexylmethylgruppen oder geradkettige oder verzweigte Alkylgruppen mit 1 bis 10 Kohlenstoffatomen, die gegebenenfalls durch eine Hydroxygruppe, eine Carboxygruppe oder eine Alkoxygruppe mit 1 bis 5 Kohlenstoffatomen, durch eine Alkoxycarbonylgruppe mit 2 bis 6 Kohlenstoffatomen, durch eine Alkylphenylgruppe mit 7 bis 16 Kohlenstoffatomen oder durch eine Alkylthien-2-yl-gruppe mit 5 bis 14 Kohlenstoffatomen substituiert sein können,

– $R_3$ ein Wasserstoffatom oder eine geradkettige oder verzweigte Alkylgruppe mit 1 bis 4 Kohlenstoffatomen,

– A eine Einfachbindung oder eine Methylengruppe oder eine Gruppe der Formel

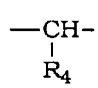

in der $R_4$ eine geradkettige oder verzweigte Alkylgruppe mit 1 bis 4 Kohlenstoffatomen darstellt,
mit der Maßgabe bedeuten, daß, wenn A eine Einfachbindung darstellt, $R_3$, $R_1$ und $R_2$ jeweils Wasserstoffatome bedeuten,

in Form des Racemats oder optischen Isomeren

und deren Additionssalze mit einer anorganischen oder organischen, pharmazeutisch annehmbaren Säure.

2. Verbindungen der allgemeinen Formel I nach Anspruch 1, worin A eine Einfachbindung bedeutet.

3. Verbindungen der allgemeinen Formel I nach Anspruch 1, worin A eine Methylengruppe bedeutet.

4. Verfahren zur Herstellung der Verbindungen der allgemeinen Formel I, **dadurch gekennzeichnet,** daß man 2,3-Dihydro-benzofuran der Formel II

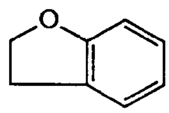

(II)

in Gegenwart eines chlorierten organischen Lösungsmittels und von Aluminiumchlorid mit einem Asparaginsäure- oder Glutaminsäure-Derivat der allgemeinen Formel (III)

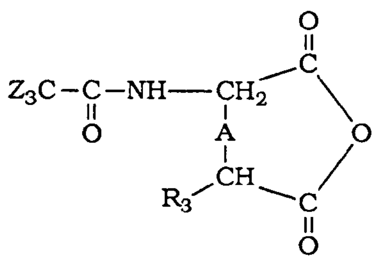

(III)

in der A und $R_3$ die oben bezüglich der allgemeinen Formel (I) angegebenen Bedeutungen besitzen und Z ein Wasserstoffatom oder ein Fluoratom bedeutet, umsetzt zur Bildung von Verbindungen der allgemeinen Formel (IV)

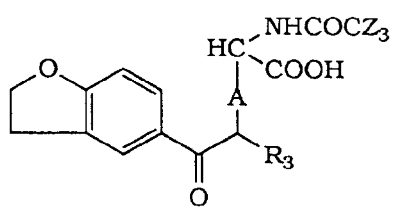

(IV)

in der $R_3$, A und Z die oben angegebenen Bedeutungen besitzen,

welche man in der Wärme und in Gegenwart von Trifluoressigsäure mit Triethylsilan reduziert zur Bildung der Verbindungen der allgemeinen Formel (V)

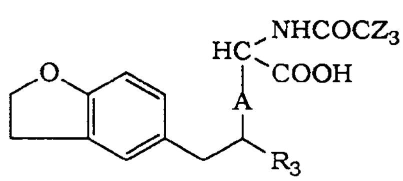

(V)

in der $R_3$, A und Z die oben angegebenen Bedeutungen besitzen,

welche man in der Wärme in Gegenwart von Phosphorsäure mit Phosphorpentoxid umsetzt zur Bildung der Verbindungen der allgemeinen Formel (VI)

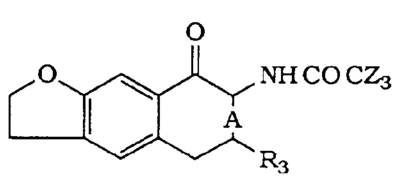

(VI)

in der $R_3$, Z und A die oben angegebenen Bedeutungen besitzen, welche man bei Raumtemperatur in saurem Medium und in Gegenwart von 5 % Palladium-auf-Kohlenstoff katalytisch hydriert zur Bildung eines Amids der allgemeinen Formel (VII)

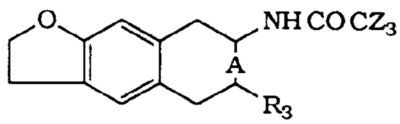 (VII)

in der $R_3$, A und Z die oben angegebenen Bedeutungen besitzen, welches man mit einer starken Base umsetzt zur Bildung einer Verbindung der allgemeinen Formel (I), in der $R_1$ und $R_2$, gleichartig sind und jeweils Wasserstoffatome bedeuten,

**welche man anschließend** alkylieren kann zur Bildung der entsprechenden sekundären oder tertiären Amine,

. oder mit einer Verbindung der allgemeinen Formel (VIII)

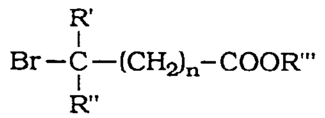 (VIII)

in der R′ und R″, die gleichartig oder verschieden sein können, jeweils Wasserstoffatome oder niedrigmolekulare Alkylgruppen mit 1 bis 4 Kohlenstoffatomen, R‴ eine niedrigmolekulare Alkylgruppe mit 1 bis 5 Kohlenstoffatomen und n eine ganze Zahl mit einem Wert von 0 bis 9 bedeuten, kondensiert zur Bildung einer Verbindung der allgemeinen Formel (I), in der $R_1$ ein Wasserstoffatom und $R_2$ eine geradkettige oder verzweigte Alkylgruppe mit 1 bis 10 Kohlenstoffatomen, welche mit einer Alkoxycarbonylgruppe mit 2 bis 6 Kohlenstoffatomen substituiert ist, bedeuten, welche man anschließend mit einer starken anorganischen Base umsetzen kann zur Bildung einer Verbindung der allgemeinen Formel (I), in der $R_1$ ein Wasserstoffatom und $R_2$ eine geradkettige oder verzweigte Alkylgruppe mit 1 bis 10 Kohlenstoffatomen, welche durch eine Carboxygruppe substituiert ist, bedeuten,

. oder mit einem Säurechlorid der allgemeinen Formel (IX)

$$W(CH_2)_nCOCl \quad (IX)$$

worin n eine ganze Zahl mit einem Wert von 0 bis 9 und W eine Phenylgruppe oder eine Thien-2-yl-gruppe bedeuten, umsetzt und anschließend die bei dieser Reaktion erhaltene Verbindung mit einem Doppelmetallhydrid reduziert zur Bildung einer Verbindung der allgemeinen Formel (I), in der $R_1$ eine Alkylgruppe mit 1 bis 10 Kohlenstoffatomen, welche durch eine Phenylgruppe oder eine Thien-2-yl-gruppe substituiert ist und $R_2$ ein Wasserstoffatom bedeuten,

. oder durch Umsetzen mit einer geeigneten Menge Formaldehyd und Ameisensäure zu Verbindungen der allgemeinen Formel I umsetzt, worin $R_3$ und $R_4$, die gleichartig sind, jeweils Methylgruppen bedeuten,

. oder mit einem Alkyliodid der allgemeinen Formel (X)

$$IR \quad (X)$$

in der R eine geradkettige oder verzweigte Alkylgruppe mit 1 bis 10 Kohlenstoffatomen (die gegebenenfalls durch eine Hydroxygruppe oder eine Alkoxygruppe mit 1 bis 5 Kohlenstoffatomen substituiert ist) oder eine Cyclohexylmethylgruppe bedeutet, in der Wärme in einem organischen Lösungsmittel in Gegenwart einer anorganischen Base

umsetzt zur Bildung der Verbindungen der allgemeinen Formel (I), in der R1 und $R_2$, die gleichartig sind, die gleichen Bedeutungen besitzen wie R,

. oder in der Wärme und in Gegenwart eines Alkohols it niedrigem Molekulargewicht mit Benzaldehyd umsetzt zur Bildung des entsprechenden Benzylimins, welches anschließend katalytisch hydriert und mit Ameisensäure und Formaldehyd umgesetzt wird zu Verbindungen der allgemeinen Formel (I), in der $R_1$ eine Methylgruppe und $R_2$ eine Cyclohexylmethylgruppe bedeuten,

. oder zunächst mit Benzaldehyd in Gegenwart eines inerten, apolaren, aromatischen Lösungsmittels und anschließend nach der Entfernung des verwendeten Lösungsmittels mit Natriumborhydrid in Gegenwart eines polaren aliphatischen Alkohols mit niedrigem Molekulargewicht umsetzt zur Bildung einer Verbindung der allgemeinen Formel (I), in der $R_1$ ein Wasserstoffatom und $R_2$ eine Benzylgruppe bedeuten,

**welche man anschließend**

. mit Formaldehyd und Ameisensäure zu Verbindungen der allgemeinen Formel (I), in der $R_1$ eine Methylgruppe und $R_2$ eine Benzylgruppe bedeuten, umsetzen kann,

. oder mit einem Alkyliodid der allgemeinen Formel (X) zu Verbindungen der allgemeinen Formel (I), in der $R_1$ die gleichen Bedeutungen besitzt wie R und $R_2$ eine Methylgruppe bedeutet, umsetzen kann,

**welche anschließend**

einer katalytischen Hydrierung zur Bildung von Verbindungen der allgemeinen Formel (I) unterworfen werden, in der $R_1$ die oben angegebene Bedeutung besitzt und $R_2$ ein Wasserstoffatom darstellt,

**welche anschließend**

mit einem Alkyliodid der allgemeinen Formel (X) umgesetzt werden können zur Bildung von Verbindungen der allgemeinen Formel (I), in der $R_1$ und $R_2$, die gleichartig oder verschieden sind, jeweils Alkylgruppen mit 1 bis 10 Kohlenstoffatomen (welche gegebenenfalls durch eine Hydroxygruppe oder eine Alkoxygruppe mit 1 bis 5 Kohlenstoffatomen substituiert sind) oder eine Cyclohexylmethylgruppe bedeuten,

welche man anschließend gewünschtenfalls mit einer anorganischen oder organischen, pharmazeutisch annehmbaren Säure in ein Salz überführen kann,

oder welche man in die optischen Isomeren auftrennen und anschließend in die Salze überführen kann.

5. Pharmazeutische Zubereitung enthaltend als Wirkstoff eine Verbindung nach einem der Ansprüche 1 bis 3, in Kombination oder in Mischung mit einem inerten, nichttoxischen, pharmazeutisch annehmbaren Trägermaterial und/oder Bindemittel.

6. Pharmazeutische Zubereitung nach Anspruch 5, enthaltend den Wirkstoff in einer Dosis von 0,5 bis 100 mg.

7. Pharmazeutische Zubereitung nach den Ansprüchen 5 und 6, enthaltend als Wirkstoff mindestens eine Verbindung nach einem der Ansprüche 1 bis 3 zur Verwendung bei der Behandlung von Erkrankungen, welche die Behandlung mit Antidepressiva, Antiaggressiva oder dopaminergen Modulatoren notwendig machen.

8. Verbindungen der allgemeinen Formel (IV)

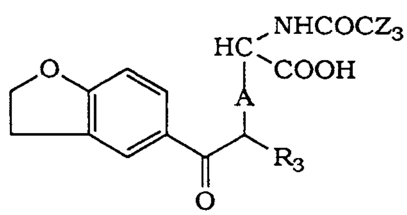

(IV)

in der

– $R_3$ ein Wasserstoffatom oder eine geradkettige oder verzweigte Alkylgruppe mit 1 bis 4 Kohlenstoffatomen,

– A eine Einfachbindung oder eine Methylengruppe oder eine Gruppe der Formel

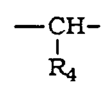

in der

– $R_4$ eine geradkettige oder verzweigte Alkylgruppe mit 1 bis 4 Kohlenstoffatomen darstellt,

und

– Z ein Wasserstoffatom oder ein Fluoratom bedeuten,

welche als Zwischenprodukte bei der Herstellung der Verbindungen der allgemeinen Formel (I) nach Anspruch 1 verwendet werden können.

9. Verbindungen der allgemeinen Formel (V)

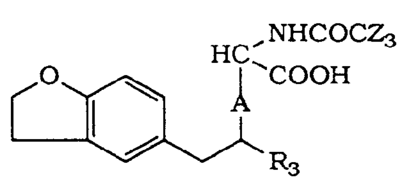

(V)

in der

- $R_3$ ein Wasserstoffatom oder eine geradkettige oder verzweigte Alkylgruppe mit 1 bis 4 Kohlenstoffatomen,
- A eine Einfachbindung, eine Methylengruppe oder eine Gruppe der Formel

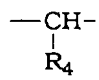

in der

- $R_4$ eine geradkettige oder verzweigte Alkylgruppe mit 1 bis 4 Kohlenstoffatomen darstellt,
  und
- Z ein Wasserstoffatom oder ein Fluoratom bedeuten,

welche als Zwischenprodukte bei der Herstellung der Verbindungen der allgemeinen Formel (I) nach Anspruch 1 verwendet werden können.